# EUROPEAN PATENT SPECIFICATION

(11) **EP 0 811 055 B1**
(45) Date of publication and mention of the grant of the patent: **09.06.2004**
(21) Application number: 95944222.9
(22) Date of filing: 21.12.1995
(51) Int. Cl.: C12N 1/06

(54) **METHOD FOR LYSING CELLS**
METHODE ZUR ZELLYSE
PROCEDE DE LYSE DE CELLULES

(43) Date of publication of application: 10.12.1997
(73) Proprietor: GENZYME CORPORATION, Cambridge, Massachusetts 02139-1562 (US)
(72) Inventor: WAN, Nick, C., Newton, MA 02166 (US); McNEILLY, David, S., Saugus, MA 01906 (US); CHRISTOPHER, Charles, W., Rockport, MA 01966 (US)
(74) Representative: Ford, Timothy James
(86) International application number: PCT/US1995/016843
(87) International publication number: WO 1997/023601

(56) References cited:
- EP-A- 0 288 425
- US-A- 4 294 824
- US-A- 4 450 103
- US-A- 4 462 940
- US-A- 4 900 677
- US-A- 4 997 932
- US-A- 5 096 818
- US-A- 5 208 160
- US-A- 5 330 914
- ENZYME AND MICROBIAL TECHNOLOGY, vol. 6, no. 7, 6 July 1984 (1984-07-06), pages 325-330, XP001008156
- JOURNAL OF CHEMICAL TECHNOLOGY AND BIOTECHNOLOGY, Volume 54, issued 1992, PAPAMICHAEL et al., "Continuous Aqueous Phase Extraction of Proteins: Automated Processing and Recycling of Process Chemicals", pages 47-55, XP000267311
- ENZYME AND MICROBIAL TECHNOLOGY, Volume 6, Number 7, issued 06 July 1984, VEIDE et al., "Continuous Extraction of Beta-D-galactosidase from Escherichia Coli in an Aqueous Two-phase System: Effects of Biomass Concentration on Partitioning and Mass Transfer", pages 325-330, XP001008156
- RODRIGUEZ et al., "Recombinant DNA Techniques: an Introduction", Published 1983, by THE BENJAMIN/CUMMINGS PUBLISHING COMPANY, INC., (CA), pages 37-46, XP002947868

## Description

### Background of the Invention

Increasing attention has been focused on the delivery of DNAs as therapeutic agents (i.e., DNA gene therapy) for the treatment of genetic diseases and for genetic immunization. Because of safety concerns with using potentially infectious viruses, researchers have been studying alternatives to viruses, using naked DNA or other non-viral methods of DNA delivery. One of the most promising non-viral methods in DNA therapy is the use of cationic lipids as delivery vehicles (Felgner, *Proc. Natl*. *Acad. Sci., 84:7413-7417, 1987*). The cationic lipids adsorb to negatively charged DNA and facilitate entry of the DNA into target cells. Successful delivery of genes via lipids into airway epithelia of rodents (Hyde, *Nature*, *362:250-255*, *1993*) have been reported. If future 'gene therapy' proves to be effective, huge quantities of plasmids or appropriate DNA will be needed. However, current methods for isolating limited amounts of DNA may impede progress in this field.

Generally, the first step in making copies of a gene of interest starts with inserting the appropriate portion of DNA into plasmids which are then replicated in "host cells". Host cells include eukaryotic and prokaryotic cells. Once a sufficient amount of plasmids are produced in the host cells, the host cells are lysed to release the internally replicated plasmids.

Numerous mechanical methods for lysing cells have been developed and published. They include pressure, cavitation, sonic or ultrasonic waves, mechanical shaking with abrasives or grinding (Sadeva, *Biopharm 7(2):26-36*, *1994*). However, few of these methods are suitable for recovery of DNA or other shear sensitive materials from cells. Chemical (Foster, *Biotechnol*. *10:273-277*, *1992*) and enzymatic (Andrews and Asenjo, *Tibtech. 5:273-277, 1987*) treatments, are more gentle and can be used for the recovery of shear sensitive products, such as DNA.

Generally, methods of plasmid isolation involve lysing the plasmid-containing cells with alkali (i.e. caustics) or enzymes in a test tube by gently inverting the tube several times. After the cells have been lysed, the cell lysate is mixed with a precipitating solution in a test tube by again gently inverting the test tube several times. The object is to precipitate contaminating cell components, e.g. genomic DNA, proteins, and protein-nucleic acid complexes, leaving the plasmids in solution. The plasmids can then be separated from the precipitate by centrifugation. While this approach can be used for small scale isolation, it is not practical for large scale production because the lysate is extremely viscous after the release of genomic DNA. Although, effective and gentle mixing of large quantities of viscous material can be achieved by using special agitators/mixers (e.g., planery mixers), these types of equipment are expensive and are not easily automated. In addition, the time of exposure of each plasmid to alkali in the large volume of solution is difficult to control and can vary a great deal and this may affect plasmid quality. Furthermore, it is crucial to handle the lysate very gently because any sheared genomic DNA may contaminate the sample and render later plasmid purification extremely difficult.

A need exists to develop an effective, economical, and automatable method for isolating high quality plasmids on a large scale to meet the future demand.

### Summary of the Invention

This invention relates to the use of static mixers in a novel manner, such as to rapidly lyse large amounts of cells. The lysis method comprises simultaneously flowing a cell suspension and a lysis solution through a static mixer, wherein the cells exit the static mixer lysed. A key advantage of the present invention is that multi-liter amounts of solution containing multi-gram amounts of cells can be lysed rapidly, making large scale biological procedures involving cell lysis feasible.

In another aspect of the present invention, the lysis method further comprises a precipitation step of simultaneously flowing the cell lysate and a precipitating solution through a second static mixer, wherein the lysate exits the static mixer with its precipitable components precipitated.

In another aspect of the present invention, the invention relates to a method for releasing intact usable plasmids from plasmid-containing cells, comprising simultaneously flowing a solution containing the plasmid-containing cells and a lysate solution through a static mixer wherein the plasmid-containing cells exit the static mixer lysed and the plasmids released. In addition, the method of releasing a plasmid further includes a preliminary step, prior to the lysing step, of simultaneously diluting the cells to be lysed to an optimal cell density by flowing the plasmid-containing cell suspension and a solution through a static mixer prior to simultaneously flowing the diluted cell suspension with a lysis solution through another static mixer (i.e., a second static mixer) with the same results. Furthermore, this method can include the precipitation step, explained above, where the cell lysate of the second static mixer is combined with a precipitating solution through a third static mixer.

### Brief Description of the Drawings

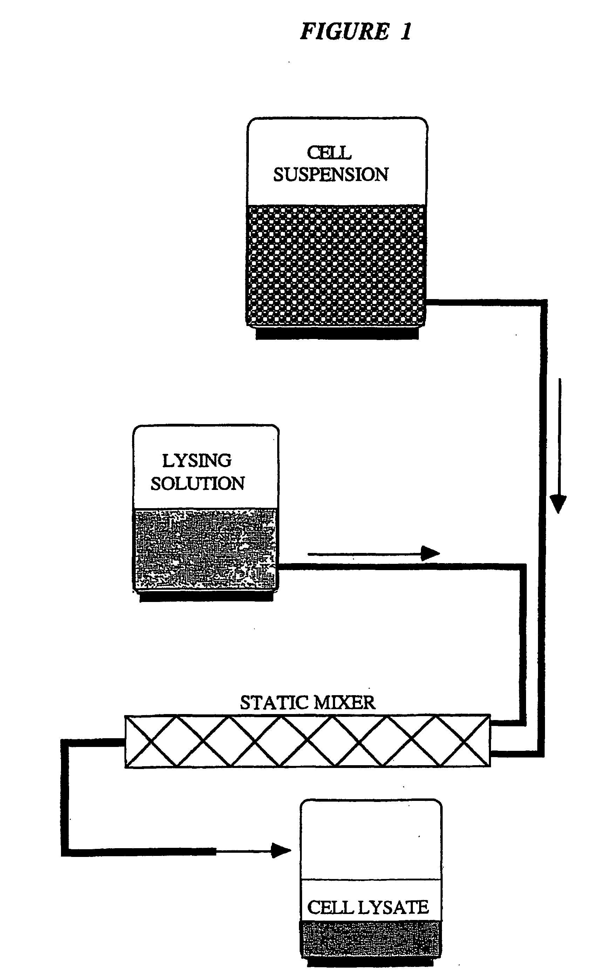

Figure 1 is an illustration of the method of the present invention where cells are passed through a static mixer simultaneously with a lysis solution, the result being that the cells exit the static mixer lysed.

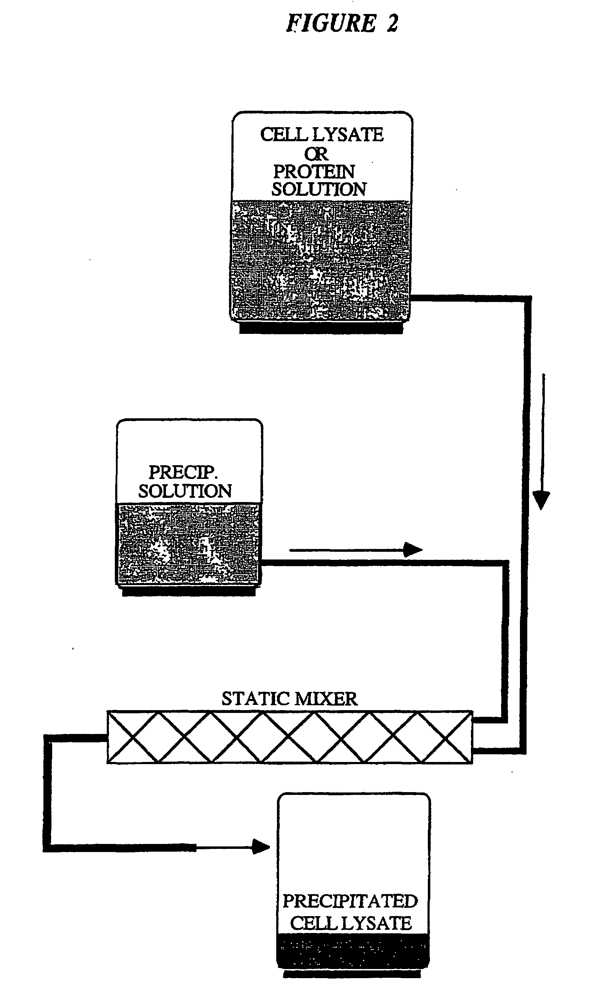

Figure 2 is an illustration of the method of the present invention where a cell lysate (or protein solution) is passed through a static mixer simultaneously with a precipitating solution the result being that the cell lysate (or protein solution) exits the static mixer precipitated.
Figure 3 is an illustration of the method of the present invention where cells are first passed through a static mixer simultaneously with a cell diluting solution, then the mixture exiting the first static mixer is passed through a second static mixer along with a lysis solution, then that mixture is passed through a third static mixer simultaneously with a precipitating solution.

### Detailed Description of the Invention

This invention is based upon the discovery that static mixers could be used to lyse cells containing plasmids, releasing the plasmids from the cells. The advantage of using such a device is that large volumes of cells can be gently and continuously lysed in-line using the static mixer and that other static mixers could be placed in-line to accomplish other functions such as dilution and precipitation. This method greatly simplifies the process of isolating plasmids from large volumes of material such that plasmid DNA is not damaged by the process. Previous methods of plasmid isolation involving caustic lysing and precipitation, which involved expensive and specialized equipment, were not practical for large scale plasmid purification.

The method of the present invention can be used to lyse any type of cell (i.e., prokaryotic or eukaryotic) for any purpose related to lysing, such as releasing desired nucleic acids or proteins from target cells to be subsequently purified. In a preferred embodiment, the method of the present invention is used to lyse host cells containing plasmids to release plasmids.

The term "lysing" refers to the action of rupturing the cell wall and/or cell membrane of a cell which is in a buffered solution (i.e., cell suspension) through chemical treatment using a solution containing a lysing agent. Lysing agents include for example, alkali, detergents, organic solvents, and enzymes. In a preferred embodiment, the lysis of cells is done to release intact plasmids from host cells. For purposes of the present invention the term "simultaneously" referring to the passage of substances through a static mixer, means that the subject substances are passing through the static mixer at approximately the same time.

For purposes of the present invention the term "flowing" refers to the passing of a liquid at a particular flow rate (e.g., liters per minute) through the static mixer, usually by the action of a pump. It should be noted that the flow rate through the static mixer is believed to affect the efficiency of lysis, precipitation and mixing.

Suitable static mixers useful in the method of the present invention are any flow through device referred to in the art as a static or motionless mixer of a length sufficient to allow the processes of the present invention. For example, for the purpose of lysing cells, the static mixer would need to have a length which would provide enough contact time between the lysing solution and the cells to cause the lysis of the subject cells during passage through the mixer. In a preferred embodiment, suitable static mixers contain an internal helical structure which causes two liquids to come in contact with one another in an opposing rotational flow causing the liquids to mix together in a turbulent flow.

For purposes of the present invention the term "precipitating" refers to the action of precipitating proteins and other cell components from a solution through chemical precipitation using a solution containing a precipitating agent. Precipitating agents include sodium dodecyl sulfate (SDS) and potassium acetate.

The term "plasmid" for purposes of the present invention include any type of replication vector which has the capability of having a non-endogenous DNA fragment inserted into it. Procedures for the construction of plasmids include those described in Maniatis *et al*., *Molecular Cloning*, *A Laboratory Manual*, *2d, Cold Spring Harbor Laboratory Press* (1989).

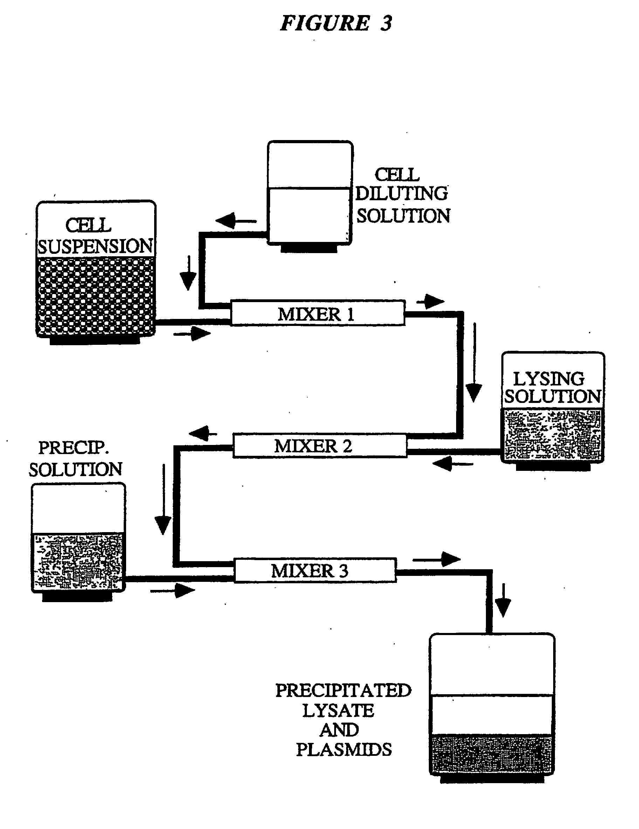

In a preferred embodiment, illustrated in Figure 3, the method of releasing plasmids from plasmid-containing host cells, comprises first simultaneously flowing the media containing the plasmid-containing host cells with a buffered solution through a static mixer i.e., the first static mixer. The mixture that exits the first static mixer is thereby equilibrated with the buffer. The buffered mixture is then simultaneously flowed through a second static mixer along with a lysis solution which, while passing through the length of the static mixer, lyses the cells releasing the plasmid and cellular components. The cell lysate is then passed through a third static mixer along with a precipitation buffer which precipitates most of the cellular components but not the plasmids.

### EXEMPLIFICATION

*Escherichia coli* (*E*. *coli*) cells that had been grown in a nutrient medium to a high cell density, the culture was then diluted directly by flowing the cells through a 1/2" x 27", 32-element, Kenics static mixer (purchased from Chemineer, N. Andover MA) along with a resuspending solution (50mM Tris/HCl, 10mM EDTA, 100 mg RNaseA/ml, pH 8.0) until the exiting cells had an optical density of 25-40 at 600nm, as read by a spectrophotometer.

The diluted cell suspension exiting the first static mixture was then flowed through a second static mixer (same type of mixer as above) along with a lysis solution (200mM NaOH, 1% weight/volume SDS, ) at a rate of 100-1,300 ml/min. The cells exited the second static mixer lysed (i.e., cell lysate).

The cell lysate exiting the second static mixer was then flowed through a third static mixer (same type as above) with a precipitating solution (2.6 M potassium acetate, pH 5.2) at a similar flow rate as passed through the second static mixer. The exiting suspension contained precipitated *E. coli* genomic DNA, proteins, and insoluble debris and the soluble plasmids. About 45 liters of mixture was collected from the third static mixer and processed. Approximately 1 gram of plasmid was isolated from this run.

All of the static mixers above were connected in series by tubing and the respective solutions were flowed or pumped though the static mixers by peristaltic pumps (Cole-Parmer, Chicago IL).

## Claims

1. A method of lysing cells comprising simultaneously flowing a cell suspension and a lysis solution through a static mixer, wherein the cells exit the static mixer lysed.

2. The method of claim 1 wherein the cells are plasmid-containing cells.

3. A method as claimed in claim 2, comprising simultaneously flowing a suspension containing the plasmid-containing cells and a lysis solution through a static mixer wherein the plasmid-containing cells exit the static mixer lysed and plasmids released from the cells.

4. A method as claimed in claim 3, comprising a preceding step of simultaneously flowing a suspension containing the plasmid-containing cells and a buffered solution through a first static mixer;

5. A method as claimed in claim 3 or 4, comprising the further step of simultaneously flowing the product of claim 3 or claim 4 through a further static mixer with a precipitating solution wherein the cellular components exit the static mixer precipitated, leaving the plasmid in solution.

6. The method of any of the preceding claims wherein the lysis solution is a solution containing a lysis agent selected from the group consisting of an alkali, a detergent, an organic solvent, and an enzyme or mixture thereof.

7. The method of claim 5 wherein the precipitating solution is a solution containing a precipitating agent selected from the group consisting of sodium dodecyl sulfate and potassium acetate or mixture thereof.

## Patentansprüche

1. Methode zur Zellyse umfassend das gleichzeitige Hindurchleiten einer Zellsuspension und einer Lyselösung durch einen statischen Mischer, wobei die Zellen den statischen Mischer im lysierten Zustand verlassen.

2. Methode nach Anspruch 1, wobei es sich bei den Zellen um plasmidhaltige Zellen handelt.

3. Methode nach Anspruch 2 umfassend das gleichzeitige Hindurchleiten einer die plasmidhaltigen Zellen enthaltenden Suspension und einer Lyselösung durch einen statischen Mischer, wobei die plasmidhaltigen Zellen den statischen Mischer im lysierten Zustand verlassen und die Plasmide aus den Zellen freigesetzt sind.

4. Methode nach Anspruch 3 umfassend einen vorhergehenden Schritt des gleichzeitigen Hindurchleitens einer die plasmidhaltigen Zellen enthaltenden Lösung und einer gepufferten Lösung durch einen ersten statischen Mischer.

5. Methode nach Anspruch 3 oder 4 umfassend den weiteren Schritt des gleichzeitigen Hindurchleitens des Produkts aus Anspruch 3 oder 4 durch einen weiteren statischen Mischer zusammen mit einer ausfällenden Lösung, wobei die zellulären Komponenten den statischen Mischer im ausgefällten Zustand verlassen unter Zurücklassen des Plasmids in Lösung.

6. Methode nach einem der vorhergehenden Ansprüche, wobei es sich bei der Lyselösung um eine Lösung handelt, die ein Lysemittel enthält, das aus der Gruppe ausgewählt ist, die aus einem Alkali, einem Detergenz, einem organischen Lösungsmittel, und einem Enzym oder einer Mischung derselben besteht.

7. Methode nach Anspruch 5, wobei es sich bei der ausfällenden Lösung um eine Lösung handelt, die ein Ausfällungsmittel enthält, das aus der Gruppe ausgewählt ist, die aus Natriumdodecylsulfat und Kaliumacetat oder einer Mischung derselben besteht.

## Revendications

1. Procédé de lyse de cellules comprenant l'écoulement simultané d'une suspension cellulaire et d'une solution de lyse au travers d'un mélangeur statique, dans lequel les cellules sortent du mélangeur statique lysées.

2. Procédé suivant la revendication 1, dans lequel les cellules sont des cellules contenant des plasmides.

3. Procédé suivant la revendication 2, comprenant l'écoulement simultané d'une suspension contenant des cellules contenant des plasmides et d'une solution de lyse au travers d'un mélangeur statique dans lequel les cellules contenant des plasmides sortent du mélangeur statique lysées et les plasmides libérés des cellules.

4. Procédé suivant la revendication 3, comprenant une étape précédente comprenant l'écoulement simultané d'une suspension contenant les cellules contenant des plasmides et d'une solution tamponnée au travers d'un premier mélangeur statique.

5. Procédé suivant la revendication 3 ou 4, comprenant l'étape supplémentaire comprenant l'écoulement simultané du produit de la revendication 3 ou 4 au travers d'un autre mélangeur statique avec une solution de précipitation dans lequel les composants cellulaires sortent du mélangeur statique précipités, en laissant le plasmide en solution.

6. Procédé suivant l'une quelconque des revendications précédentes, dans lequel la solution de lyse est une solution contenant un agent de lyse sélectionné parmi le groupe comprenant un alkali, un détergent, un solvant organique et un enzyme ou un mélange de ceux-ci.

7. Procédé suivant la revendication 5, dans lequel la solution de précipitation est une solution contenant un agent de précipitation sélectionné parmi le groupe comprenant du dodécylsulfate de sodium et de l'acétate de potassium ou un mélange de ceux-ci.
